# EUROPÄISCHE PATENTSCHRIFT

(11) **EP 3 094 740 B1**
(45) Veröffentlichungstag und Bekanntmachung des Hinweises auf die Patenterteilung: **11.09.2019**
(21) Anmeldenummer: 14799822.3
(22) Anmeldetag: 20.11.2014
(51) Int. Cl.: C12Q 1/68, B01L 3/00

(54) **ANALYSEEINHEIT ZUM DURCHFÜHREN EINER VERSCHACHTELTEN POLYMERASEKETTENREAKTION, ANALYSEVORRICHTUNG, VERFAHREN ZUM BETREIBEN EINER SOLCHEN ANALYSEEINHEIT UND VERFAHREN ZUM HERSTELLEN EINER SOLCHEN ANALYSEEINHEIT**
ANALYSIS UNIT FOR PERFORMING A NESTED POLYMERASE CHAIN REACTION, ANALYSIS DEVICE, METHOD FOR OPERATING AN ANALYSIS UNIT OF SAID TYPE, AND METHOD FOR MANUFACTURING AN ANALYSIS UNIT OF SAID TYPE
UNITÉ D'ANALYSE PERMETTANT LA RÉALISATION D'UNE AMPLIFICATION EN CHAÎNE PAR POLYMÉRASE NICHÉE, DISPOSITIF D'ANALYSE, PROCÉDÉ POUR FAIRE FONCTIONNER UNE TELLE UNITÉ D'ANALYSE ET PROCÉDÉ DE FABRICATION D'UNE TELLE UNITÉ D'ANALYSE

(30) Priorität: 14.01.2014 DE 102014200509
(43) Veröffentlichungstag der Anmeldung: 23.11.2016
(73) Patentinhaber: Robert Bosch GmbH, 70442 Stuttgart (DE)
(72) Erfinder: HOFFMANN, Jochen, 71229 Leonberg (DE)
(86) Internationale Anmeldenummer: PCT/EP2014/075141
(87) Internationale Veröffentlichungsnummer: WO 2015/106858

(56) Entgegenhaltungen:
- WO-A1-2008/106719
- WO-A2-2008/122002
- DE-A1- 10 315 074
- US-A1- 2005 019 792
- R. PEYTAVI ET AL: "Microfluidic Device for Rapid (<15 min) Automated Microarray Hybridization", CLINICAL CHEMISTRY, Bd. 51, Nr. 10, 1. Oktober 2005 (2005-10-01), Seiten 1836-1844, XP055104033, ISSN: 0009-9147, DOI: 10.1373/clinchem.2005.052845
- ROBIN HUI LIU ET AL: "SELF-CONTAINED, FULLY INTEGRATED BIOCHIP FOR SAMPLE PREPARATION, POLYMERASE CHAIN REACTION AMPLIFICATION, AND DNA MICROARRAY DETECTION", ANALYTICAL CHEMISTRY, AMERICAN CHEMICAL SOCIETY, US, Bd. 76, Nr. 7, 1. April 2004 (2004-04-01), Seiten 1824-1831, XP001196720, ISSN: 0003-2700, DOI: 10.1021/AC0353029
- ZHANG C ET AL: "Survey and Summary : Miniaturized PCR chips for nucleic acid amplification and analysis : latest advances and future trends", NUCLEIC ACIDS RESEARCH, OXFORD UNIVERSITY PRESS, GB, Bd. 35, Nr. 13, 1. Juli 2007 (2007-07-01), Seiten 4223-4237, XP008133567, ISSN: 0305-1048, DOI: 10.1093/NAR/GKM389 [gefunden am 2007-06-18]

## Beschreibung

### Stand der Technik

Der hier vorgestellte Ansatz bezieht sich auf eine Analyseeinheit zum Durchführen einer verschachtelten Polymerasekettenreaktion, eine Analysevorrichtung zum Betreiben einer solchen Analyseeinheit, ein Verfahren zum Betreiben einer solchen Analyseeinheit und ein Verfahren zum Herstellen einer solchen Analyseeinheit.

Bei einer verschachtelten Polymerasekettenreaktion, auch nested-PCR genannt (PCR bezeichnet; PCR = Polymerase Chain Reaction = engl. Polymerasekettenreaktion), werden zwei Polymerasekettenreaktionen nacheinander durchgeführt. Ein PCR-Produkt einer ersten Polymerasekettenreaktion kann hierbei auf mehrere neue Reaktionskammern verteilt und mit jeweils verschiedenen PCR-Primern versetzt werden. Da die neuen Primerpärchen auf einem erzeugten DNA-Abschnitt weiter innen binden, entsteht bei einer zweiten Polymerasekettenreaktion ein kürzeres PCR-Produkt als bei der ersten Polymerasekettenreaktion. Somit werden in der zweiten Polymerasekettenreaktion nur DNA-Moleküle der ersten Polymerasekettenreaktion amplifiziert, die diese zweiten Primerbindungsstellen enthalten. Dadurch werden unspezifische PCR-Produkte aus der ersten Reaktion nicht weiter amplifiziert. Ferner lassen sich mit diesem Verfahren verschiedene Zielsequenzen spezifisch nachweisen.

Bei einem konventionellen Nukleinsäurenachweis kann eine erste Polymerasekettenreaktion in einem PCR-Cycler durchgeführt werden und ein PCR-Produkt der ersten Polymerasekettenreaktion händisch auf Reaktionskammern für eine zweite Polymerasekettenreaktion verteilt werden.

Bei einem Nukleinsäurenachweis mittels eines Lab-on-a-chip-Systems, auch LOC genannt, kann ein zu untersuchendes Probenmaterial zunächst mittels einer Polymerasekettenreaktion amplifiziert und anschließend auf einem Microarray analysiert werden.

### Offenbarung der Erfindung

Vor diesem Hintergrund werden mit dem hier vorgestellten Ansatz eine Analyseeinheit zum Durchführen einer verschachtelten Polymerasekettenreaktion, eine Analysevorrichtung zum Betreiben einer solchen Analyseeinheit, ein Verfahren zum Betreiben einer solchen Analyseeinheit sowie schließlich ein Verfahren zum Herstellen einer solchen Analyseeinheit gemäß den Hauptansprüchen vorgestellt. Vorteilhafte Ausgestaltungen ergeben sich aus den jeweiligen Unteransprüchen und der nachfolgenden Beschreibung.

Der vorliegende Ansatz schafft eine Analyseeinheit zum Durchführen einer verschachtelten Polymerasekettenreaktion, wobei die Analyseeinheit folgende Merkmale aufweist:
zumindest eine Vorbereitungskammer zur Durchführung einer ersten Polymerasekettenreaktion, um ein Reaktionsprodukt zu erhalten; und
zumindest eine Auswertestruktur zur Durchführung einer zweiten Polymerasekettenreaktion unter Verwendung des Reaktionsprodukts, wobei die Auswertestruktur mit der Vorbereitungskammer fluidisch gekoppelt oder koppelbar ist und wobei die Auswertestruktur zumindest zwei Segmentkammern umfasst, die fluidisch parallel geschaltet sind.

Unter einer Vorbereitungskammer kann ein in der Analyseeinheit ausgebildeter Hohlraum zum Aufnehmen eines vorzubereitenden Fluids verstanden werden. Beispielsweise kann die Vorbereitungskammer zumindest zwei fluidisch gekoppelte oder koppelbare Teilkammern zum Durchführen verschiedener Reaktionsschritte der ersten Polymerasekettenreaktion aufweisen. Hierbei können die Teilkammern beispielsweise fluidisch in Reihe geschaltet sein. Unter einem vorzubereitenden Fluid kann ein Fluid mit einem biochemischen Material wie etwa Nukleinsäuren verstanden werden. Vorbestimmte Teilabschnitte des biochemischen Materials können mittels der ersten Polymerasekettenreaktion, etwa durch Beimischen eines entsprechenden Reagenzes, vervielfältigt werden, um ein für eine nachfolgende Auswertung benötigtes Fluid oder Reaktionsprodukt zu erhalten. Unter einer Auswertestruktur (die auch als Auswertekammer bezeichnet werden kann) kann ein in der Analyseeinheit ausgebildeter weiterer Hohlraum zum Aufnehmen des (benötigten) Fluids oder Reaktionsproduktes verstanden werden. Unter einer Segmentkammer kann ein Teilbereich der Auswertestruktur verstanden werden, in dem die zweite Polymerasekettenreaktion oder zumindest ein Teilschritt der zweiten Polymerasekettenreaktion erfolgen kann. Unter einer Auswertung kann eine Erkennung eines Vorhandenseins eines vorbestimmten Materials und/oder eine Erkennung einer Quantität und/oder einer Qualität des vorbestimmten Materials verstanden werden.

Der vorliegende Ansatz beruht auf der Erkenntnis, dass es möglich ist, eine verschachtelte Polymerasekettenreaktion auf einem mikrofluidischen Chip durchzuführen. Hierbei kann der Chip zumindest zwei fluidisch miteinander verbundene Reaktionsräume umfassen, die eine räumliche Trennung verschiedener hintereinander ausgeführter Polymerasekettenreaktionen ermöglichen. Ein für eine Auswertung vorgesehener Reaktionsraum kann hierbei zumindest zwei Segmentkammern aufweisen, wodurch verschiedene Nachweise vorbestimmter Abschnitte eines biochemischen Materials zeitgleich durchgeführt werden können.

Ein solcher Chip bietet den Vorteil, dass ein zu analysierendes Fluid oder Reaktionsprodukt ohne manuelles Eingreifen von dem einen in den anderen Reaktionsraum verbracht werden kann. Dadurch können beispielsweise DNA-Kontaminationen oder Pipettierfehler vermieden werden und Personalkosten für spezialisierte Laborkräfte gespart werden. Durch eine kompakte Anordnung der Reaktionsräume auf dem Chip können im Vergleich zu herkömmlichen Lösungen eine kürzere Prozesszeit sowie eine vereinfachte Prozessführung erreicht werden. Zudem kann ein solcher Chip sehr kosteneffizient in großen Stückzahlen hergestellt werden.

Gemäß einer Ausführungsform des vorliegenden Ansatzes können die Segmentkammern je zumindest ein Reagenz zum Durchführen der zweiten Polymerasekettenreaktion aufweisen. Unter einem Reagenz kann beispielsweise ein Primer- und/oder Sondenmaterial verstanden werden, das bei Kontakt mit einem vorbestimmten Teilabschnitt eines biochemischen Materials eine Polymerasekettenreaktion zum Vervielfältigen und/oder Identifizieren des Teilabschnitts bewirkt. Dadurch können je nach Art des verwendeten Reagenzes verschiedene biochemische Analysen sehr zuverlässig und flexibel durchgeführt werden.

Hierbei können die Segmentkammern je eine andere Menge des Reagenzes und/oder je zumindest ein anderes Reagenz aufweisen. Dies bietet den Vorteil, dass eine Mehrzahl unterschiedlicher biochemischer Nachweise gleichzeitig durchgeführt werden kann, wodurch sich eine Prozesszeit deutlich verkürzen lässt. Dadurch, dass jede Nachweisreaktion in einer eigenen Kammer stattfindet, können Ungenauigkeiten bei einer Auswertung der Nachweisreaktionen reduziert werden.

Gemäß einer weiteren Ausführungsform des vorliegenden Ansatzes können ein Teil der Vorbereitungskammer und ein weiterer Teil der Vorbereitungskammer ausgebildet sein, um auf je eine andere Temperatur gebracht zu werden. Alternativ oder zusätzlich können ein Teil der Auswertestruktur und ein weiterer Teil der Auswertestruktur ausgebildet sein, um auf je eine andere Temperatur gebracht zu werden. Alternativ oder zusätzlich können ein Bereich der Vorbereitungskammer und ein Bereich der Auswertestruktur ausgebildet sein, um auf je eine andere Temperatur gebracht zu werden. Dadurch können die zum Durchführen einzelner Reaktionsschritte der ersten und der zweiten Polymerasekettenreaktion erforderlichen Temperaturen mit hoher Flexibilität bereitgestellt werden, was eine Vielzahl unterschiedlicher biochemischer Nachweise auf der Analyseeinheit ermöglicht.

Ferner umfasst die Auswertestruktur zumindest zwei weitere Segmentkammern. Hierbei ist je eine der weiteren Segmentkammern mit je einer der Segmentkammern fluidisch in Reihe geschaltet. Dies bietet den Vorteil, dass bestimmte Teilreaktionen der zweiten Polymerasekettenreaktion räumlich und chemisch getrennt voneinander durchgeführt werden können. Beispielsweise kann hierbei ein Reaktionsvolumen zwischen den Segmentkammern und den weiteren Segmentkammern hin und her geschoben werden, um eine zweischrittige Polymerasekettenreaktion durchzuführen.

Des Weiteren können die Segmentkammern und die weiteren Segmentkammern ausgebildet sein, um auf je eine andere Temperatur gebracht zu werden. Dadurch können platzsparend zwei unterschiedliche Temperaturzonen zum Durchführen einer zweischrittigen zweiten Polymerasekettenreaktion realisiert werden.

Gemäß einer weiteren Ausführungsform des vorliegenden Ansatzes kann eine Mehrzahl von Segmentkammern zumindest teilweise ringförmig um die Vorbereitungskammer angeordnet sein. Durch eine solche Anordnung der Segmentkammern kann die Analyseeinheit besonders kompakt ausgeführt werden.

Ferner können die Vorbereitungskammer und die Auswertestruktur Komponenten einer Kanalstruktur zum Steuern eines Fluidstroms in der Analyseeinheit sein. Insbesondere kann hierbei die Kanalstruktur zumindest ein Ventil aufweisen. Das Ventil kann insbesondere ausgebildet sein, um die Vorbereitungskammer und die Auswertestruktur fluidisch zu entkoppeln. Unter einer Kanalstruktur kann eine Anordnung von mit der Vorbereitungs- und/oder der Auswertestruktur verbundenen Kanälen in der Analyseeinheit verstanden werden. Ferner kann die Kanalstruktur eine fluidische Verbindung mit einer Außenumgebung der Analyseeinheit ermöglichen. Bei einem Ventil kann es sich beispielsweise um eine auslenkbare, fluiddichte Membran handeln. Mittels der Kanalstruktur kann eine vorbestimmte Menge des Fluids kontrolliert durch einzelne Reaktionsbereiche der Vorbereitungs- und/oder der Auswertestruktur geleitet werden, um die verschachtelte Polymerasekettenreaktion durchzuführen.

Gemäß einer weiteren Ausführungsform des vorliegenden Ansatzes kann die Analyseeinheit zumindest eine Pumpkammer zum Pumpen eines vorzubereitenden Fluids in die Vorbereitungskammer und/oder des Fluids aus der Vorbereitungskammer aufweisen. Unter einer Pumpkammer kann ein Hohlraum der Analyseeinheit verstanden werden, der mit der Vorbereitungskammer fluidisch gekoppelt oder koppelbar ist. Beispielsweise kann die Pumpkammer ausgebildet sein, um mit einem vorbestimmten Druck beaufschlagt zu werden. Dadurch kann eine kontrollierte Befüllung und/oder Entleerung der Vorbereitungskammer sichergestellt werden.

Der vorliegende Ansatz schafft ferner eine Analysevorrichtung zum Betreiben einer Analyseeinheit gemäß einer der vorangehend beschriebenen Ausführungsformen. Hierbei weist die Analysevorrichtung folgende Merkmale auf:
ein Halteelement zum Platzieren und/oder Halten der Analyseeinheit während des Betreibens der Analyseeinheit;
eine Temperiereinheit zum Temperieren eines Fluids in der von dem Halteelement gehaltenen Analyseeinheit, insbesondere wobei die Temperiereinheit ausgebildet ist, um zeitgleich unterschiedliche Abschnitte der Analyseeinheit auf je eine andere Temperatur zu bringen; und
eine Auswerteeinheit zum Auswerten der zweiten Polymerasekettenreaktion unter Verwendung des Fluids und/oder zumindest eines Produkts der zweiten Polymerasekettenreaktion unter Verwendung des Fluids.

Unter einem Halteelement kann beispielsweise auch eine Auflagefläche verstanden werden, auf der die Analyseeinheit während des Betriebs der Analysevorrichtung platziert ist. Unter einer Temperiereinheit kann eine Einheit verstanden werden, die zumindest einen Teilabschnitt der Analyseeinheit erhitzt oder kühlt. Unter einer Auswerteeinheit kann eine Einheit verstanden werden, die eine Zustandsveränderung eines Reaktionsvolumens während der zweiten Polymerasekettenreaktion erfasst, beispielsweise durch optische Analyse einer von dem Reaktionsvolumen konvertierten oder abgegebenen elektromagnetischen Strahlung. Eine solche Ausführungsform des vorliegenden Ansatzes bietet den Vorteil einer sehr kompakten Analyse eines biochemischen Materials mit einer kostengünstig und einfach bereitzustellenden Analyseeinheit.

Zudem schafft der vorliegende Ansatz ein Verfahren zum Betreiben einer Analyseeinheit gemäß einer der vorangehend beschriebenen Ausführungsformen, wobei das Verfahren folgende Schritte umfasst:
Bereitstellen der Analyseeinheit;
Beaufschlagen der Vorbereitungskammer der Analyseeinheit mit einem vorzubereitenden Fluid und/oder mit einem Reagenz zum Durchführen einer ersten Polymerasekettenreaktion in der Vorbereitungskammer;
Verbringen eines Produkts der ersten Polymerasekettenreaktion in die Auswertestruktur der Analyseeinheit; und
Auswerten einer zweiten Polymerasekettenreaktion unter Verwendung des Produkts der ersten Polymerasekettenreaktion in der Auswertestruktur und/oder zumindest eines Produkts der zweiten Polymerasekettenreaktion unter Verwendung des Produkts der ersten Polymerasekettenreaktion.

Schließlich schafft der vorliegende Ansatz ein Verfahren zum Herstellen einer Analyseeinheit gemäß einer der vorangehend beschriebenen Ausführungsformen, wobei das Verfahren folgende Schritte umfasst:
Bereitstellen zumindest eines Deckelelements und eines Bodenelements, wobei das Deckelelement zumindest zwei Deckelausnehmungen und das Bodenelement zumindest zwei Bodenausnehmungen aufweist; und
Bilden eines Schichtverbunds mit zumindest einer Vorbereitungskammer zur Durchführung einer ersten Polymerasekettenreaktion, um ein Reaktionsprodukt zu erhalten, und zumindest einer Auswertestruktur zur Durchführung einer zweiten Polymerasekettenreaktion unter Verwendung des Reaktionsprodukts, wobei das Bilden durch Anordnen der Deckelausnehmungen gegenüberliegend den Bodenausnehmungen erfolgt, wobei die Auswertestruktur mit der Vorbereitungskammer fluidisch gekoppelt oder koppelbar ist und wobei die Auswertestruktur zumindest zwei Segmentkammern umfasst, die fluidisch parallel geschaltet sind.

Unter einem Deckelelement und/oder einem Bodenelement kann beispielsweise eine Komponente verstanden werden, die aus einem Kunststoff, insbesondere aus einem Polymer, gefertigt ist. Unter einer Ausnehmung kann beispielsweise eine Vertiefung in dem Deckelelement oder dem Bodenelement verstanden werden. Unter einem Schichtverbund kann eine Kombination miteinander verbundener Schichten verstanden werden.

Der hier vorgestellte Ansatz schafft ferner eine Steuereinheit oder ein Steuergerät, das ausgebildet ist, um die Schritte einer Variante eines hier vorgestellten Verfahrens in entsprechenden Einrichtungen durchzuführen bzw. umzusetzen. Auch durch diese Ausführungsvariante der Erfindung in Form eines Steuergeräts kann die der Erfindung zugrunde liegende Aufgabe schnell und effizient gelöst werden.

Unter einer Steuereinheit oder einem Steuergerät kann vorliegend ein elektrisches Gerät verstanden werden, das Sensorsignale verarbeitet und in Abhängigkeit davon Steuer- und/oder Datensignale ausgibt. Die Steuereinheit und/oder das Steuergerät kann eine Schnittstelle aufweisen, die hard- und/oder softwaremäßig ausgebildet sein kann. Bei einer hardwaremäßigen Ausbildung können die Schnittstellen beispielsweise Teil eines sogenannten System-ASICs sein, der verschiedenste Funktionen des Steuergeräts beinhaltet. Es ist jedoch auch möglich, dass die Schnittstellen eigene, integrierte Schaltkreise sind oder zumindest teilweise aus diskreten Bauelementen bestehen. Bei einer softwaremäßigen Ausbildung können die Schnittstellen Softwaremodule sein, die beispielsweise auf einem Mikrocontroller neben anderen Softwaremodulen vorhanden sind.

Von Vorteil ist auch ein Computerprogrammprodukt oder Computerprogramm mit Programmcode, der auf einem maschinenlesbaren Träger oder Speichermedium wie einem Halbleiterspeicher, einem Festplattenspeicher oder einem optischen Speicher gespeichert sein kann und zur Durchführung und/oder Ansteuerung der Schritte des Verfahrens nach einer der vorstehend beschriebenen Ausführungsformen verwendet wird, insbesondere wenn das Programmprodukt auf einem Computer oder einer Vorrichtung ausgeführt wird.

Der hier vorgestellte Ansatz wird nachstehend anhand der beigefügten Zeichnungen beispielhaft näher erläutert. Es zeigen:
- Fig. 1: eine schematische Darstellung einer Analyseeinheit gemäß einem Ausführungsbeispiel der vorliegenden Offenbarung;
- Fig. 2: eine schematische Darstellung einer verschachtelten Polymerasekettenreaktion gemäß einem Ausführungsbeispiel der vorliegenden Offenbarung;
- Fig. 3: eine Aufsichtdarstellung einer Analyseeinheit gemäß einem Ausführungsbeispiel der vorliegenden Offenbarung;
- Fig. 4: eine Aufsichtdarstellung einer Analyseeinheit gemäß einem Ausführungsbeispiel der vorliegenden Offenbarung;
- Fig. 5: eine Aufsichtdarstellung einer Analyseeinheit gemäß einem Ausführungsbeispiel der vorliegenden Offenbarung;
- Fig. 6: eine Aufsichtdarstellung einer Analyseeinheit gemäß einem Ausführungsbeispiel der vorliegenden Offenbarung;
- Fig. 7: eine Querschnittsdarstellung einer Analysevorrichtung zum Betreiben einer Analyseeinheit gemäß einem Ausführungsbeispiel der vorliegenden Offenbarung;
- Fig. 8: ein Ablaufdiagramm eines Ausführungsbeispiels eines Verfahrens zum Betreiben einer Analyseeinheit; und
- Fig. 9: ein Ablaufdiagramm eines Ausführungsbeispiels eines Verfahrens zum Herstellen einer Analyseeinheit.

In der nachfolgenden Beschreibung günstiger Ausführungsbeispiele der vorliegenden Offenbarung werden für die in den verschiedenen Figuren dargestellten und ähnlich wirkenden Elemente gleiche oder ähnliche

Bezugszeichen verwendet, wobei auf eine wiederholte Beschreibung dieser Elemente verzichtet wird.

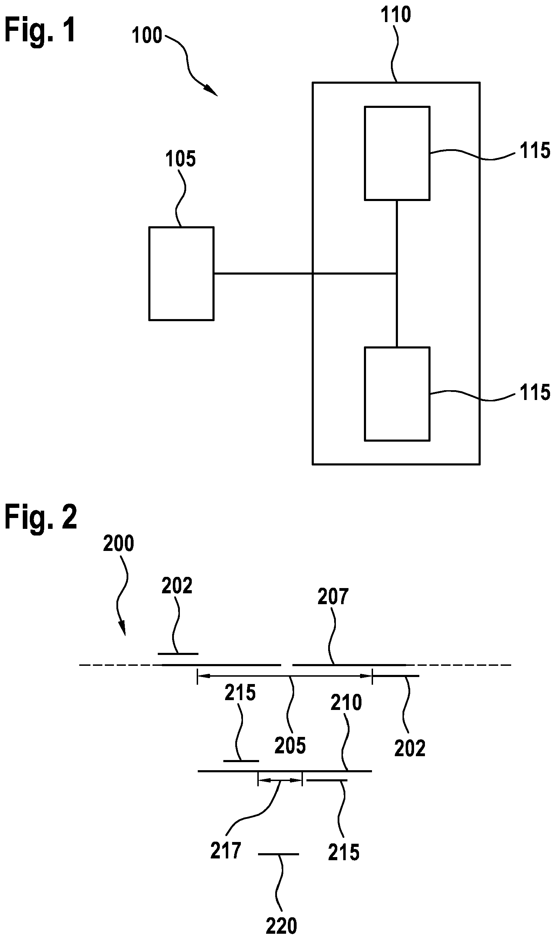

Fig. 1 zeigt eine Aufsichtdarstellung einer Analyseeinheit 100 gemäß einem Ausführungsbeispiel der vorliegenden Offenbarung. Die Analyseeinheit 100 umfasst eine Vorbereitungskammer 105 sowie eine Auswertestruktur 110, die fluidisch miteinander verbunden sind. Die Auswertestruktur 110 umfasst ferner zwei Segmentkammern 115, die fluidisch parallel geschaltet sind.

Die Vorbereitungskammer 105 ist ausgebildet, um ein Fluid mit einem biochemischen Ausgangsmaterial aufzunehmen. Hierbei dient die Vorbereitungskammer 105 als Reaktionsbereich zum Durchführen einer ersten Polymerasekettenreaktion, durch die vorbestimmte Teilabschnitte des Ausgangmaterials vervielfältigt werden, um ein für eine nachfolgende Auswertung vorbereitetes Fluid zu erhalten. Die Segmentkammern 115 sind ausgebildet, um je eine vorbestimmte Menge des vorbereiteten Fluids aufzunehmen. Hierbei dienen die Segmentkammern 115 als räumlich voneinander getrennte Reaktionsbereiche zum Durchführen einer zweiten Polymerasekettenreaktion, durch die vorbestimmte Teilbereiche der Teilabschnitte vervielfältigt und/oder identifiziert werden.

Ein wichtiger Aspekt des hier beschriebenen Ansatzes ist die Offenbarung eines mikrofluidischen Systems bestehend unter anderem aus Kanälen, Ventilen und Kavitäten in einer bestimmten Anordnung. Beispielsweise wird zunächst eine zu untersuchende Probenlösung in eine Kammer 105 eingebracht und mittels einer ersten Polymerasekettenreaktion voramplifiziert. Im Anschluss daran wird diese Probenlösung auf mehrere Kammern 115 aliquotiert, d. h. verteilt, und mit unterschiedlichen Primerpärchen vermischt. So können bei einer zweiten Polymerasekettenreaktion in jeder Kammer 115 eine oder mehrere unterschiedliche DNA-Sequenzen nachgewiesen werden. Hierdurch ergeben sich folgende Vorteile.

Die Anforderungen bezüglich eines biochemischen Designs einer Multiplex-PCR können deutlich verringert werden, da die Nachweisreaktion für jede einzelne DNA-Sequenz in einer einzigen Nachweiskammer durchgeführt wird. Es brauchen also nicht erst PCR-Reaktionsbedingungen für den Nachweis mehrerer DNA-Sequenzen in einer einzigen Reaktionskammer gefunden zu werden.

Durch Amplifikation eines längeren, mehrere Zielsequenzen umfassenden DNA-Bereichs in der ersten Polymerasekettenreaktion kann genügend Material für Einzelnachweisreaktionen im Rahmen der zweiten Polymerasekettenreaktion erzeugt werden. Wenn beispielsweise ein zu untersuchendes Probenmaterial, das nur wenige Zielmoleküle enthält, direkt auf mehrere Reaktionskammern verteilt wird, kann die Gefahr bestehen, dass einzelne Kammern zu wenig genetisches Ausgangsmaterial für eine Nachweisreaktion enthalten.

Es sind zwei Auslesevarianten möglich: Real-time-Auslesen während der zweiten Polymerasekettenreaktion oder End-point-Auslesen nach der zweiten Polymerasekettenreaktion.

Die beschriebenen Strukturen ermöglichen eine Integration und Realisierung vieler PCR-Assays bzw. Biocontents. Es gibt deutlich mehr Assays, bei denen DNA-Moleküle direkt über eine Singleplex- bis Quadplex-PCR nachgewiesen werden, als in einer Kombination aus Multiplex-PCR und anschließender Mikroarray-Analyse.

Zudem wird kein DNA-Mikroarray für den Nachweis unterschiedlicher DNA-Moleküle benötigt. Dadurch sinken auch die Anforderungen an eine optische Detektionseinheit.

Fig. 2 zeigt eine schematische Darstellung einer verschachtelten Polymerasekettenreaktion 200 gemäß einem Ausführungsbeispiel der vorliegenden Offenbarung. In einer ersten Polymerasekettenreaktion lagert sich ein erstes Primerpaar 202 an einen vorbestimmten Teilabschnitt 205 eines biochemischen Ausgangsmaterials 207 an, um ein erstes PCR-Produkt 210 als Kopie des Teilabschnitts 205 zu erhalten. In einer zweiten Polymerasekettenreaktion lagert sich ein zweites Primerpaar 215 an einen vorbestimmten Teilbereich 217 des ersten PCR-Produkts 210 an, um ein zweites PCR-Produkt 220 als Kopie des Teilbereichs 217 zu erhalten. Das zweite PCR-Produkt 220 ist somit kürzer als das erste PCR-Produkt 202 und enthält nur diejenigen biochemischen Verbindungen, die sich zwischen Bindungsstellen von Primern des zweiten Primerpaares 215 befinden. Dabei sollten die PCR-Produkte 210 und 220 die beiden Primerpaare mit enthalten (d. h. länger sein) und nicht nur den jeweils inneren Bereich.

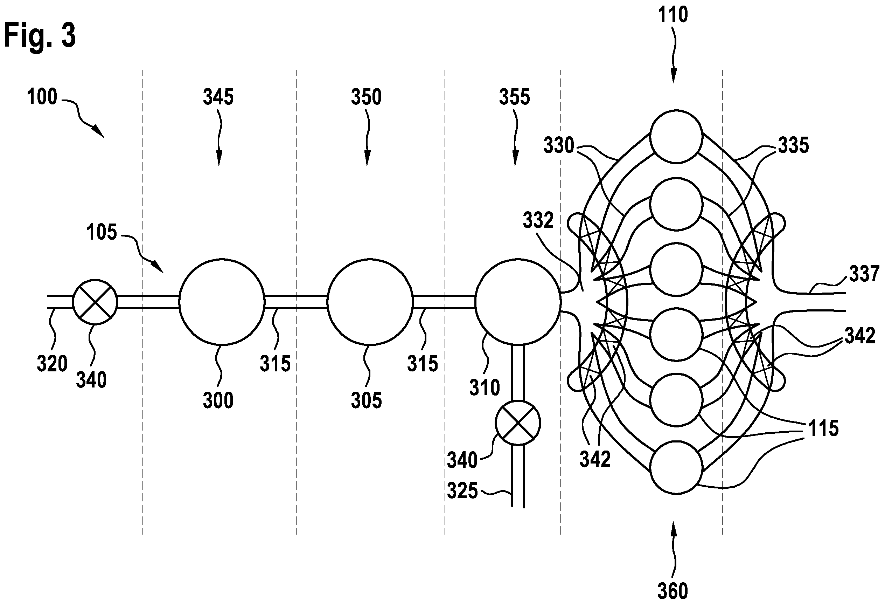

Fig. 3 zeigt eine Aufsichtdarstellung einer Analyseeinheit 100 gemäß einem Ausführungsbeispiel der vorliegenden Offenbarung. Die Vorbereitungskammer 105 ist mit einer ersten Teilkammer 300, einer zweiten Teilkammer 305 und einer dritten Teilkammer 310 ausgeführt. Die Teilkammern 300, 305, 310 sind über zwei Kanäle 315 fluidisch in Reihe geschaltet, wobei die zweite Teilkammer 305 zwischen der ersten Teilkammer 300 und der dritten Teilkammer 310 angeordnet ist. Die Auswertestruktur 110 ist beispielhaft mit sechs Segmentkammern 115 ausgeführt, die fluidisch parallel geschaltet sind. Die Segmentkammern 115 und die Teilkammern 300, 305, 310 sind je entlang einer gemeinsamen Achse angeordnet, wobei eine Achse der Segmentkammern 115 quer zu einer Achse der Teilkammern 300, 305, 310 verläuft, sodass sich eine T-förmige Anordnung ergibt.

Die erste Teilkammer 300 ist mit einem Einleitkanal 320 zum Einleiten eines Fluids in die erste Teilkammer 300 ausgeführt. Die dritte Teilkammer 310 ist mit einem Ausleitkanal 325 zum Ausleiten des Fluids aus der dritten Teilkammer 310 ausgebildet. Zusätzlich ist die dritte Teilkammer 310 über Verbindungskanäle 330 mit den Segmentkammern 115 fluidisch gekoppelt. Die Verbindungskanäle 330 laufen in einem gemeinsamen Verbindungskanal 332 zusammen, der angrenzend an die dritte Teilkammer 310 angeordnet ist. Die Segmentkammern 115 sind über Auslasskanäle 335 fluidisch mit einer Außenumgebung der Analyseeinheit 100 gekoppelt. Die Auslasskanäle 335 laufen in einem gemeinsamen Auslasskanal 337 zusammen, der beispielsweise in einem der Außenumgebung zugewandten Bereich der Analyseeinheit 100 ausgebildet ist. Hierbei verlaufen der Einleitkanal 320, die Kanäle 315, der gemeinsame Verbindungskanal 332 und der gemeinsame Auslasskanal 337 beispielsweise entlang einer gemeinsamen Achse.

In dem Einleitkanal 320 und dem Ausleitkanal 325 ist je ein Vorbereitungskammerventil 340, in den Verbindungskanälen 330 und den Auslasskanälen 335 je ein Auswertestrukturventil 342 ausgebildet, um einen Fluidstrom in der Analyseeinheit 100 zu steuern.

Die erste Teilkammer 300 ist in einer ersten Temperaturzone 345, die zweite Teilkammer 305 in einer zweiten Temperaturzone 350 und die dritte Teilkammer 310 in einer dritten Temperaturzone 355 der Analyseeinheit 100 angeordnet. Die Temperaturzonen 345, 350, 355 sind ausgebildet, um auf je eine andere vorbestimmte konstante Temperatur gebracht zu werden. Die Segmentkammern 115 sind in einer variablen Temperaturzone 360 der Analyseeinheit 100 angeordnet, deren Temperatur zwischen vorbestimmten Werten variierbar ist.

Gemäß diesem Ausführungsbeispiel ist neben den Temperaturzonen 345, 350, 355 als konstant temperierten Zonen ein regelbares Temperierelement wie beispielsweise ein Peltier-Element für einen Bereich 360 vorgesehen, der zwischen den für eine Polymerasekettenreaktion notwendigen Temperaturen alterniert.

Die Teilkammern 300, 305, 310 sind ausgebildet, um eine dreischrittige erste Polymerasekettenreaktion durchzuführen. Hierbei wird ein Probefluid von der ersten Temperaturzone 345 über die zweite Temperaturzone 350 in die dritte Temperaturzone 355 verbracht. Die Segmentkammern 115 sind ausgebildet, um eine zweischrittige zweite Polymerasekettenreaktion durchzuführen. Hierzu wird das Probefluid nach der ersten Polymerasekettenreaktion von den Teilkammern 300, 305, 3410 in die Segmentkammern 115 geleitet und abwechselnd auf unterschiedliche vorbestimmte Temperaturen gebracht.

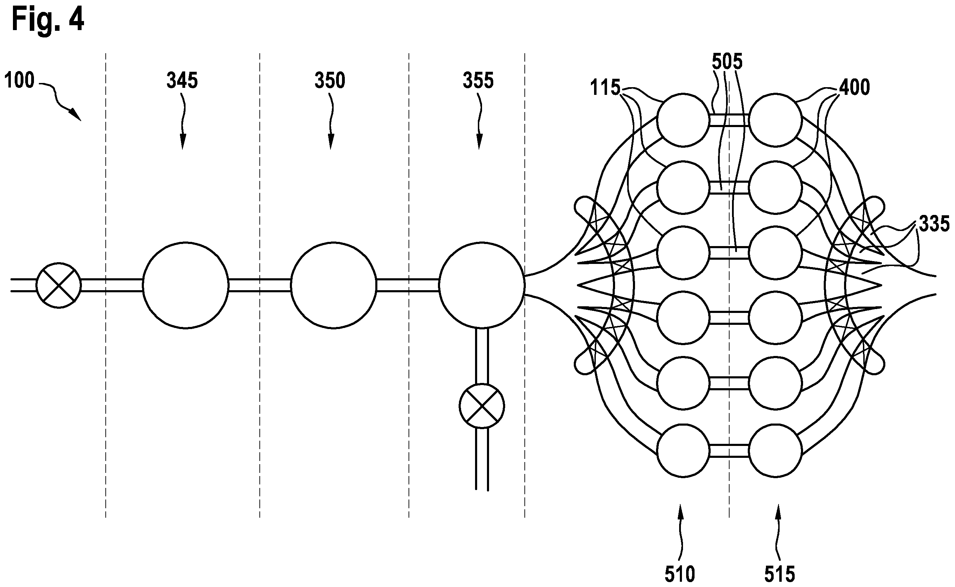

Fig. 4 zeigt eine Aufsichtdarstellung einer Analyseeinheit 100 gemäß einem Ausführungsbeispiel der vorliegenden Offenbarung. Im Unterschied zu Fig. 3 ist die in Fig. 4 gezeigte Analyseeinheit 100 mit sieben Segmentkammern 115 realisiert. Die sieben Segmentkammern 115 sind je mit einer weiteren Segmentkammer 400 über einen Segmentverbindungskanal 505 fluidisch parallel geschaltet. Die Auslasskanäle 335 sind mit den weiteren Segmentkammern 400 fluidisch gekoppelt.

Die Segmentkammern 115 sind auf einer ersten Temperaturzone 510 und die weiteren Segmentkammern 400 auf einer zweiten Temperaturzone 515 der Analyseeinheit 100 angeordnet, wobei die Temperaturzonen 510, 515 ausgebildet sind, um auf je eine andere konstante Temperatur gebracht zu werden.

Gemäß einem Ausführungsbeispiel der vorliegenden Offenbarung werden die Reaktionsvolumina für die zweite Polymerasekettenreaktion zwischen zwei auf unterschiedlichen Temperaturbereichen befindlichen Kammern 115,400 hin und her geschoben. Diese Ausführungsform bietet den Vorteil, dass kein Peltier-Element benötigt wird, dafür aber zwei weitere Temperaturzonen 510, 515 neben den Temperaturzonen 345, 350, 355 für die erste Polymerasekettenreaktion.

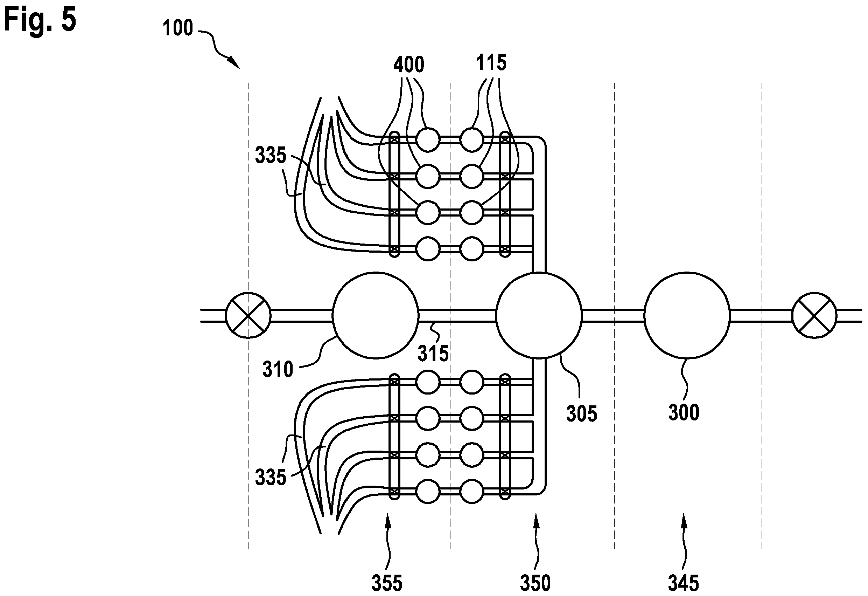

Fig. 5 zeigt eine Aufsichtdarstellung einer Analyseeinheit 100 gemäß einem Ausführungsbeispiel der vorliegenden Offenbarung. Im Gegensatz zu Fig. 4 weist die Analyseeinheit 100 in Fig. 5 je acht Segmentkammern 115 und acht weitere Segmentkammern 400 auf. Hierbei sind die Segmentkammern 115 in der zweiten Temperaturzone 350 und die weiteren Segmentkammern 400 in der dritten Temperaturzone 355 angeordnet. Die Segmentkammern 115 und die weiteren Segmentkammern 400 sind beiderseits des Kanals 315 je in Vierergruppen angeordnet, wobei die Segmentkammern 115 über die Verbindungskanäle 330 mit der zweiten Teilkammer 305 fluidisch verbunden sind. Ferner laufen beiderseits des Kanals 315 je vier der acht Auslasskanäle 335 in einem gemeinsamen Kanal zusammen, um eine fluidische Verbindung mit der Außenumgebung der Analyseeinheit 100 zu ermöglichen.

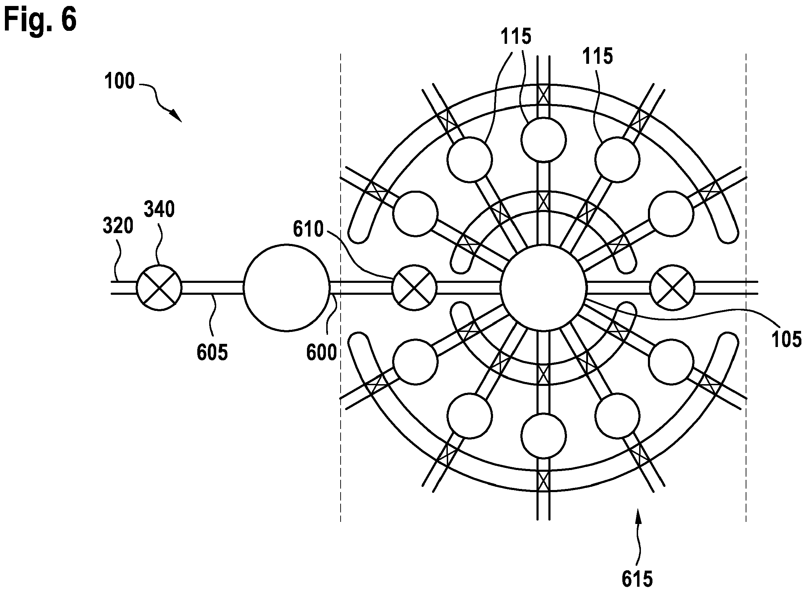

Gemäß einem Ausführungsbeispiel der vorliegenden Offenbarung erfolgt bei einer dreischrittigen ersten Polymerasekettenreaktion eine Anlagerung der Primer in der Kammer 300, eine Verlängerung in der Kammer 305 und eine Denaturierung in der Kammer 310. Die Kammern 115, 400 für die zweite Polymerasekettenreaktion liegen je auf den Temperaturzonen 350, 355, um eine zweischrittige Polymerasekettenreaktion durchzuführen. Ein Primerdesign ist so gewählt, dass die Primer der zweiten Polymerasekettenreaktion bei der Verlängerungstemperatur von 72 °C binden. Dies hat erstens den Vorteil, dass eine zweischrittige zweite Polymerasekettenreaktion durchgeführt werden kann. Dadurch können eine größere Zeitersparnis und eine höhere Spezifität erreicht werden. Zweitens sind die Primer der ersten Polymerasekettenreaktion ausgebildet, um bei dieser Temperatur nicht mehr zu binden. Daher kann nur die gewünschte zweite Polymerasekettenreaktion ablaufen. Drittens werden drei Temperaturzonen 345, 350, 355 benötigt. Fig. 6 zeigt eine Aufsichtdarstellung einer Analyseeinheit 100 gemäß einem Ausführungsbeispiel der vorliegenden Offenbarung. Die in Fig. 5 gezeigte Analyseeinheit 100 weist beispielhaft zehn Segmentkammern 115 auf, die kreisförmig um die Vorbereitungskammer 105 angeordnet sind. Hierbei sind je fünf Segmentkammern 115 halbkreisförmig auf einander gegenüberliegenden Seiten der Vorbereitungskammer 105 angeordnet. Im Gegensatz zu den vorangehend beschriebenen Figuren laufen die Auslasskanäle 335 in Fig. 6 nicht in einem gemeinsamen Auslasskanal zusammen, sondern sind als Einzelkanäle strahlenförmig um die Vorbereitungskammer 105 angeordnet. Die Segmentkammern 115 und die Vorbereitungskammer 105 sind in einer gemeinsamen Temperaturzone 615 angeordnet, deren Temperatur zwischen den zum Durchführen der erste und zweiten Polymerasekettenreaktion erforderlichen Werten variierbar ist.

Die Vorbereitungskammer 105 ist über einen Pumpkanal 600 mit einer Pumpkammer 605 zum Pumpen des Fluids in die Vorbereitungskammer 105 fluidisch gekoppelt. Im Pumpkanal 600 ist ein Pumpventil 310 ausgebildet, um ein Fluid kontrolliert von der Pumpkammer 605 in die Vorbereitungskammer 105 und/oder von der Vorbereitungskammer 105 in die Pumpkammer 605 zu leiten. An die Pumpkammer 605 ist ferner der Einleitkanal 320 mit dem Vorbereitungskammerventil 340 angeschlossen.

Das Reaktionsvolumen für die erste Polymerasekettenreaktion wird mit der Pumpkammer 605 in die Reaktionskammer 105, auch Vorbereitungskammer 105 genannt, befördert. Diese Anordnung hat den Vorteil, dass ein Platzverbrauch minimiert wird und nur ein einziges Temperierelement, beispielsweise ein Peltier-Element, benötigt wird.

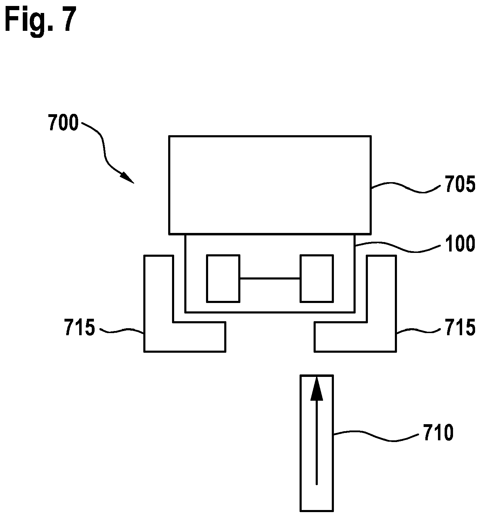

Fig. 7 zeigt eine Querschnittsdarstellung einer Analysevorrichtung 700 zum Betreiben einer Analyseeinheit 100 gemäß einem Ausführungsbeispiel der vorliegenden Offenbarung. Die Analysevorrichtung 700 ist ausgebildet, um eine nested-PCR auf einem polymeren Mehrschichtsystem durchzuführen. Ein solches Lab-on-a-chip-System ermöglicht die Durchführung hintereinander geschalteter Polymerasekettenreaktionen und damit einen spezifischen und parallelen Nachweis multipler DNA-Sequenzen.

Die Analysevorrichtung 700 umfasst eine Temperiereinheit 705, die beispielhaft auf einer Oberseite der Analyseeinheit 100 angebracht ist. Auf einer der Oberseite gegenüberliegenden Unterseite der Analyseeinheit 100 ist eine Auswerteeinheit 710 zum Auswerten der zweiten Polymerasekettenreaktion und/oder zumindest eines Produkts der zweiten Polymerasekettenreaktion vorgesehen. Die Anordnung aus Temperiereinheit 705 und Auswerteeinheit 710 kann auch umgekehrt sein. Die Analyseeinheit 100 ist ferner in einem Halteelement 715 angeordnet, das ausgebildet ist, um die Analyseeinheit 100 in der Analysevorrichtung 700 zwischen der Temperiereinheit 705 und der Auswerteeinheit 710 zu fixieren. Das Halteelement 715 weist beispielsweise eine rahmenähnliche Struktur auf, in die die Analyseeinheit 100 eingeschoben oder hineingelegt werden kann.

Das Temperierelement 710 umfasst beispielsweise mehrere Teileinheiten, die ausgebildet sind, um verschiedene benachbart zueinander angeordnete Temperaturzonen der Analyseeinheit 100 zeitgleich auf unterschiedliche Temperaturen zu bringen.

Thermische Energie wird beispielsweise durch Verwendung von Heizfingern oder transparenten Heizfolien in definierte Bereiche eines mikrofluidischen Chips, auch Analyseeinheit 100 genannt, eingebracht. Transparente Heizfolien haben den Vorteil, dass Fluoreszenzsignale der Reaktionskammern 115 während der Polymerasekettenreaktion ausgelesen werden können (real-time PCR). Die Konstanttemperatur-Heizelemente sind ein- oder beidseitig an dem Chip angebracht. Werden Peltier-Elemente verwendet, so werden diese beispielsweise von einer Seite an dem Chip angebracht. Von einer anderen Seite des Chips kann dann in Echtzeit ausgelesen werden.

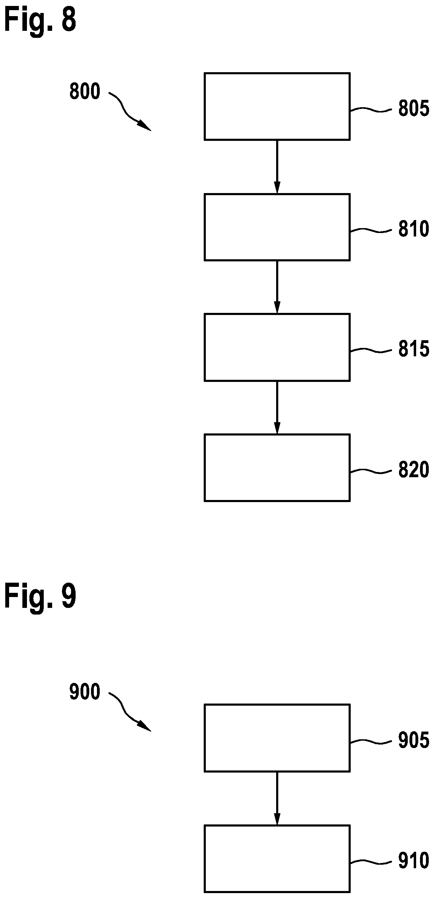

Fig. 8 zeigt ein Ablaufdiagramm eines Ausführungsbeispiels eines Verfahrens 800 zum Betreiben einer Analyseeinheit. Das Verfahren 800 umfasst einen Schritt 805 des Bereitstellens der Analyseeinheit. In einem weiteren Schritt 810 wird die Vorbereitungskammer der Analyseeinheit mit einem vorzubereitenden Fluid und/oder mit einem Reagenz zum Durchführen einer ersten Polymerasekettenreaktion in der Vorbereitungskammer beaufschlagt. Anschließend erfolgt ein Schritt 815 des Verbringens eines Produkts der ersten Polymerasekettenreaktion in die Auswertestruktur der Analyseeinheit. Schließlich erfolgt ein Schritt 820 des Auswertens einer zweiten Polymerasekettenreaktion in der Auswertestruktur und/oder zumindest eines Produkts der zweiten Polymerasekettenreaktion.

Gemäß einem in Fig. 3 gezeigten Ausführungsbeispiel der vorliegenden Offenbarung besteht ein fluidisches System aus einem mikrofluidischen Kanal als Einleitkanal 320, durch den die Kammern 300, 305, 310, 115 zunächst mit einer biologisch inaktiven Flüssigkeit zur Verdrängung von Luft und Benetzung der Kammern befüllt werden. Anschließend werden die Kammern 300, 305, 310, 115 durch Auslenkung einer an einer Oberseite der Kammern 300, 305, 310, 115 befindlichen Membran entleert. Die Ventile 340, 342 werden verschlossen. Nun werden die Ventile 340 geöffnet und die drei PCR-Kammern 300, 305, 310, auch Teilkammern 300, 305, 310 genannt, mit einem PCR-Reaktionsmix beladen, der alle Reaktionskomponenten für eine erste Polymerasekettenreaktion enthält. Zwei der drei Kammern 300, 305, 310 werden wieder entleert und die Ventile 340 werden geschlossen. Durch zyklisches Verschieben eines Reaktionsvolumens zwischen den Kammern 300, 305, 310 wird eine dreischrittige Polymerasekettenreaktion durchgeführt. Die Kammern 300, 305, 310 befinden sich hierbei auf den für eine Polymerasekettenreaktion benötigten Temperaturen von 50 °C bis 65 °C (Anlagerungstemperatur), 72 °C (Verlängerungstemperatur) und 95 °C (Aufschmelztemperatur). Nach der ersten Polymerasekettenreaktion wird das Reaktionsvolumen in die Kammer 310 überführt; das der Kammer 310 zugewandte Ventil 342 wird geöffnet. Die in die Kammern 115 gedrückten Membranen werden gleichzeitig angehoben, wodurch das Reaktionsvolumen in die Kammern gesaugt wird (Aliquotierung). Das der Kammer 310 zugewandte Ventil 342 wird wieder verschlossen. In den Kammern 115 sind jeweils unterschiedliche Primer und Sonden vorgelagert, die von dem eingeleiteten Reaktionsvolumen aufgenommen werden. Dadurch entstehen in jeder einzelnen Kammer 115 Reaktionsbedingungen für einen Nachweis unterschiedlicher DNA-Sequenzen in der zweiten Polymerasekettenreaktion.

Die Primer und Sonden für die zweite Polymerasekettenreaktion können direkt in den Kammern 115, 400 vorgelagert sein. Hierfür können alle aus dem Stand der Technik bekannten Verfahren angewendet werden, wie etwa Lypophilisierung, Verwendung von DNAstable oder Gelifizierung.

Bei einer nested-PCR sollten die Primer der ersten Polymerasekettenreaktion in der zweiten Polymerasekettenreaktion inaktiv sind. Zum einen können die Primer der ersten Polymerasekettenreaktion Uracil anstatt Thymin enthalten. Führt man vor der zweiten Polymerasekettenreaktion eine enzymatische Reaktion mit dem Enzym Uracil-Glykosylase durch, auch als UNG-Verdau bezeichnet, so werden ausschließlich uracilhaltige Primer inaktiviert. Zum anderen können die Schmelztemperaturen so gewählt sein, dass die Primer der ersten Polymerasekettenreaktion eine niedrigere Schmelztemperatur als die Primer der zweiten Polymerasekettenreaktion aufweisen.

Bei der zweiten Polymerasekettenreaktion können pro Reaktionskammer n mehrere DNA-Sequenzen m, typischerweise eins bis vier, durch Verwendung unterschiedlich markierter Sonden nachgewiesen werden. Dadurch erhöht sich die Anzahl an nachweisbaren DNA-Sequenzen um den Faktor n mal m.

Fig. 9 zeigt ein Ablaufdiagramm eines Ausführungsbeispiels eines Verfahrens 900 zum Herstellen einer Analyseeinheit. Das Verfahren 900 umfasst einen Schritt 905 des Bereitstellens zumindest eines Deckelelements und eines Bodenelements. Hierbei weist das Deckelelement zumindest zwei Deckelausnehmungen und das Bodenelement zumindest zwei Bodenausnehmungen auf. Ferner umfasst das Verfahren einen Schritt 905 des Bildens eines Schichtverbunds mit zumindest einer Vorbereitungskammer zur Durchführung einer ersten Polymerasekettenreaktion, um ein Reaktionsprodukt, und zumindest einer zur Durchführung einer zweiten Polymerasekettenreaktion unter Verwendung des Reaktionsprodukts. Hierbei erfolgt das Bilden durch Anordnen der Deckelausnehmungen gegenüberliegend den Bodenausnehmungen, wobei die Auswertestruktur mit der Vorbereitungskammer fluidisch gekoppelt oder koppelbar ist und wobei die Auswertestruktur zumindest zwei Segmentkammern umfasst, die fluidisch parallel geschaltet sind.

Die beispielhaft dargestellten benötigten Strukturen in den Polymersubstraten werden beispielsweise durch Fräsen, Spritzguss, Heißprägen oder Laserstrukturierung erzeugt.

Als Materialien für ein Polymersubstrat eignen sich Thermoplaste wie beispielsweise Polycarbonat (PC), Polypropylen (PP), Polyethylen (PE), Polymethylmethacrylat (PMMA), Cyclo-Polefin-Polymer (COP) oder Cyclo-Polefin-Copolymer (COC)

Als Materialien für eine Polymermembran eignen sich beispielsweise Elastomer, thermoplastisches Elastomer wie thermoplastisches Urethan (TPU) oder thermoplastisches Styrol (TPS), Thermoplaste, Heißklebefolien oder Siegelfolien für Mikrotiterplatten.

Eine Dicke des Polymersubstrats beträgt beispielsweise 0,5 mm bis 5 mm.

Ein Durchmesser eines Kanals in den Polymersubstraten beträgt beispielsweise 10 µm bis 3 mm.

Eine Dicke der Polymermembran beträgt beispielsweise 5 µm bis 500 µm.

Ein Volumen von Kavitäten in den Polymersubstraten beträgt beispielsweise 1 mm³ bis 1000 mm³.

In einem beispielhaften Prozessablauf ist ein Parameterraum der ersten Polymerasekettenreaktion folgendermaßen festgelegt: 1 bis 20 Thermozyklen, Temperaturzyklen mit 93 °C bis 96 °C für die Denaturierung, mit 72 °C für die Verlängerung und mit 54 °C bis 65 °C für die Primeranbindung. Es ist auch möglich, die Schritte auf der Verlängerungstemperatur wegzulassen, um eine zweischrittige Polymerasekettenreaktion durchzuführen.

Ein Parameterraum der zweiten Polymerasekettenreaktion ist beispielsweise folgendermaßen festgelegt: 20 bis 60 Thermozyklen, Temperaturzyklen mit 93 °C bis 96 °C für die Denaturierung, mit 72 °C für die Verlängerung und mit 54 °C bis 65 °C für die Primeranbindung. Auch hier ist es möglich, die Schritte auf der Verlängerungstemperatur wegzulassen, um eine zweischrittige Polymerasekettenreaktion durchzuführen.

Die erste Polymerasekettenreaktion umfasst beispielsweise 1 bis 10 Reaktionen. Die zweite Polymerasekettenreaktion umfasst beispielsweise 1 bis 50 Reaktionen.

Die beschriebenen und in den Figuren gezeigten Ausführungsbeispiele sind nur beispielhaft gewählt. Unterschiedliche Ausführungsbeispiele können vollständig oder in Bezug auf einzelne Merkmale miteinander kombiniert werden. Auch kann ein Ausführungsbeispiel durch Merkmale eines weiteren Ausführungsbeispiels ergänzt werden.

Ferner können die hier vorgestellten Verfahrensschritte wiederholt sowie in einer anderen als in der beschriebenen Reihenfolge ausgeführt werden.

Umfasst ein Ausführungsbeispiel eine "und/oder"-Verknüpfung zwischen einem ersten Merkmal und einem zweiten Merkmal, so ist dies so zu lesen, dass das Ausführungsbeispiel gemäß einer Ausführungsform sowohl das erste Merkmal als auch das zweite Merkmal und gemäß einer weiteren Ausführungsform entweder nur das erste Merkmal oder nur das zweite Merkmal aufweist.

## Patentansprüche

1. Analyseeinheit (100) zum Durchführen einer verschachtelten Polymerasekettenreaktion (200), wobei die Analyseeinheit (100) folgende Merkmale aufweist:
zumindest eine Vorbereitungskammer (105) zur Durchführung einer ersten Polymerasekettenreaktion, um ein Reaktionsprodukt zu erhalten; und
zumindest eine Auswertestruktur (110) zur Durchführung einer zweiten Polymerasekettenreaktion unter Verwendung des Reaktionsprodukts, wobei die Auswertestruktur (110) mit der Vorbereitungskammer (105) fluidisch gekoppelt oder koppelbar ist und wobei die Auswertestruktur (110) zumindest zwei Segmentkammern (115) umfasst, die fluidisch parallel geschaltet sind,
wobei die Vorbereitungskammer (105) mit einer ersten Teilkammer (300), einer zweiten Teilkammer (305) und einer dritten Teilkammer (310) ausgeführt ist, wobei die erste Teilkammer (300) in einer ersten Temperaturzone (345), die zweite Teilkammer (305) in einer zweiten Temperaturzone (350) und die dritte Teilkammer (310) in einer dritten Temperaturzone (355) der Analyseeinheit (100) für die Durchführung einer dreischrittigen ersten Polymerasekettenreaktion angeordnet ist,
wobei die zumindest zwei Segmentkammern (115) je mit einer weiteren Segmentkammer (400) fluidisch für eine zweischrittige zweite Polymerasekettenreaktion verbunden sind, wobei die Segmentkammern (115) mit der zweiten Teilkammer (305) fluidisch verbunden sind und wobei die Segmentkammern (115) in der zweiten Temperaturzone (350) und die weiteren Segmentkammern (400) in der dritten Temperaturzone (355) angeordnet sind.

2. Analyseeinheit (100) gemäß Anspruch 1, **dadurch gekennzeichnet, dass** die Segmentkammern (115) je zumindest ein Reagenz zum Durchführen der zweiten Polymerasekettenreaktion aufweisen.

3. Analyseeinheit (100) gemäß Anspruch 2, **dadurch gekennzeichnet, dass** die Segmentkammern (115) je eine andere Menge des Reagenzes und/oder je zumindest ein anderes Reagenz aufweisen.

4. Analyseeinheit (100) gemäß einem der vorangegangenen Ansprüche, **dadurch gekennzeichnet, dass** ein Teil der Vorbereitungskammer (105) und ein weiterer Teil der Vorbereitungskammer (105) ausgebildet sind, um auf je eine andere Temperatur gebracht zu werden, und/oder ein Teil der Auswertestruktur (110) und ein weiterer Teil der Auswertestruktur (110) ausgebildet sind, um auf je eine andere Temperatur gebracht zu werden, und/oder ein Bereich der Vorbereitungskammer (105) und ein Bereich der Auswertestruktur (110) ausgebildet sind, um auf je eine andere Temperatur gebracht zu werden.

5. Analyseeinheit (100) gemäß einem der vorangegangenen Ansprüche, **dadurch gekennzeichnet, dass** die Auswertestruktur (110) zumindest zwei weitere Segmentkammern (400) umfasst, wobei je eine der weiteren Segmentkammern (400) mit je einer der Segmentkammern (115) fluidisch in Reihe geschaltet ist.

6. Analyseeinheit (100) gemäß Anspruch 5, **dadurch gekennzeichnet, dass** die Segmentkammern (115) und die weiteren Segmentkammern (400) ausgebildet sind, um auf je eine andere Temperatur gebracht zu werden.

7. Analyseeinheit (100) gemäß einem der vorangegangenen Ansprüche, **dadurch gekennzeichnet, dass** die Vorbereitungskammer (105) und die Auswertestruktur (110) Komponenten einer Kanalstruktur zum Steuern eines Fluidstroms in der Analyseeinheit (100) sind, insbesondere wobei die Kanalstruktur zumindest ein Ventil (340, 342) aufweist, insbesondere wobei das Ventil (342) ausgebildet ist, um die Vorbereitungskammer (105) und die Auswertestruktur (110) fluidisch zu entkoppeln.

8. Analyseeinheit (100) gemäß einem der vorangegangenen Ansprüche, **gekennzeichnet durch** zumindest eine Pumpkammer (605) zum Pumpen eines vorzubereitenden Fluids in die Vorbereitungskammer (105) und/oder des Reaktionsproduktes aus der Vorbereitungskammer (105).

9. Verfahren (800) zum Betreiben einer Analyseeinheit (100) gemäß einem der Ansprüche 1 bis 8, wobei das Verfahren (800) folgende Schritte umfasst:
Bereitstellen (805) der Analyseeinheit (100);
Beaufschlagen (810) der Vorbereitungskammer (105) der Analyseeinheit (100) mit einem vorzubereitenden Fluid und/oder mit einem Reagenz zum Durchführen einer dreischrittigen ersten Polymerasekettenreaktion in der die erste Teilkammer (300), die zweite Teilkammer (305) und die dritte Teilkammer (310) umfassenden Vorbereitungskammer (105);
Verbringen (815) eines Produkts der ersten Polymerasekettenreaktion in die Auswertestruktur (110) der Analyseeinheit (100); und
Auswerten (820) einer in den Segmentkammern (115) und den damit fluidisch verbundenen weiteren Segmentkammern durchgeführten zweischrittige zweiten Polymerasekettenreaktion unter Verwendung des Produkts der ersten Polymerasekettenreaktion in der Auswertestruktur (110) und/oder zumindest eines Produkts der zweiten Polymerasekettenreaktion unter Verwendung des Produkts der ersten Polymerasekettenreaktion.

10. Verfahren (900) zum Herstellen einer Analyseeinheit (100) gemäß einem der Ansprüche 1 bis 8, wobei das Verfahren (900) folgende Schritte umfasst:
Bereitstellen (905) zumindest eines Deckelelements und eines Bodenelements, wobei das Deckelelement zumindest zwei Deckelausnehmungen und das Bodenelement zumindest zwei Bodenausnehmungen aufweist; und
Bilden (910) eines Schichtverbunds mit zumindest einer Vorbereitungskammer (105) zur Durchführung einer dreischrittigen ersten Polymerasekettenreaktion, um ein Reaktionsprodukt zu erhalten, wobei die Vorbereitungskammer (105) mit einer ersten Teilkammer (300) in einer ersten Temperaturzone (345), einer zweiten Teilkammer (305) in einer zweiten Temperaturzone (350) und einer dritten Teilkammer (310) in einer dritten Temperaturzone (355) ausgeführt ist, und zumindest einer Auswertestruktur (110) zur Durchführung einer zweischrittigen zweiten Polymerasekettenreaktion unter Verwendung des Reaktionsprodukts, wobei das Bilden (910) durch Anordnen der Deckelausnehmungen gegenüberliegend den Bodenausnehmungen erfolgt, wobei die Auswertestruktur (110) mit der Vorbereitungskammer (105) fluidisch gekoppelt oder koppelbar ist und wobei die Auswertestruktur (110) zumindest zwei Segmentkammern (115) umfasst, die fluidisch parallel geschaltet und je mit einer weiteren Segmentkammer (400) fluidisch verbunden und wobei die Segmentkammern (115) in der zweiten Temperaturzone (350) und die weiteren Segmentkammern (400) in der dritten Temperaturzone (355) angeordnet werden.

11. Steuereinheit, die ausgebildet ist, um alle Schritte eines Verfahrens gemäß Anspruch 9 durchzuführen, umzusetzen und/oder anzusteuern.

12. Computerprogramm, das dazu eingerichtet ist, alle Schritte eines Verfahrens gemäß Anspruch 9 durchzuführen, umzusetzen und/oder anzusteuern.

13. Maschinenlesbares Speichermedium mit einem darauf gespeicherten Computerprogramm nach Anspruch 12.

## Claims

1. Analysis unit (100) for performing a nested polymerase chain reaction (200), wherein the analysis unit (100) has the following features:
at least one preparation chamber (105) for performing a first polymerase chain reaction, in order to obtain a reaction product; and
at least one evaluation structure (110) for performing a second polymerase chain reaction using the reaction product, wherein the evaluation structure (110) is or can be fluidically coupled to the preparation chamber (105), and wherein the evaluation structure (110) comprises at least two segment chambers (115), which are fluidically connected in parallel,
wherein the preparation chamber (105) is designed with a first subsidiary chamber (300), a second subsidiary chamber (305) and a third subsidiary chamber (310), wherein the first subsidiary chamber (300) is arranged in a first temperature zone (345), the second subsidiary chamber (305) is arranged in a second temperature zone (350), and the third subsidiary chamber (310) is arranged in a third temperature zone (355) of the analysis unit (100), for performing a three-step first polymerase chain reaction,
wherein the at least two segment chambers (115) are each fluidically connected to a further segment chamber (400) for a two-step second polymerase chain reaction, wherein the segment chambers (115) are fluidically connected to the second subsidiary chamber (305), and wherein the segment chambers (115) are arranged in the second temperature zone (350) and the further segment chambers (400) are arranged in the third temperature zone (355).

2. Analysis unit (100) according to Claim 1, **characterized in that** the segment chambers (115) each have at least one reagent for performing the second polymerase chain reaction.

3. Analysis unit (100) according to Claim 2, **characterized in that** the segment chambers (115) each have a different quantity of the reagent and/or each have at least one different reagent.

4. Analysis unit (100) according to one of the preceding claims, **characterized in that** a part of the preparation chamber (105) and a further part of the preparation chamber (105) are designed to be brought each to a different temperature, and/or a part of the evaluation structure (110) and a further part of the evaluation structure (110) are designed to be brought each to a different temperature, and/or a region of the preparation chamber (105) and a region of the evaluation structure (110) are designed to be brought each to a different temperature.

5. Analysis unit (100) according to one of the preceding claims, **characterized in that** the evaluation structure (110) comprises at least two further segment chambers (400), wherein in each case one of the further segment chambers (400) is fluidically connected in series to in each case one of the segment chambers (115).

6. Analysis unit (100) according to Claim 5, **characterized in that** the segment chambers (115) and the further segment chambers (400) are designed to be brought each to a different temperature.

7. Analysis unit (100) according to one of the preceding claims, **characterized in that** the preparation chamber (105) and the evaluation structure (110) are components of a channel structure for controlling a fluid stream in the analysis unit (100), in particular wherein the channel structure has at least one valve (340, 342), in particular wherein the valve (342) is designed to fluidically decouple the preparation chamber (105) and the evaluation structure (110).

8. Analysis unit (100) according to one of the preceding claims, **characterized by** at least one pump chamber (605) by which a fluid to be prepared is pumped into the preparation chamber (105) and/or the reaction product is pumped out of the preparation chamber (105).

9. Method (800) for operating an analysis unit (100) according to one of Claims 1 to 8, wherein the method (800) comprises the following steps:
providing (805) the analysis unit (100);
supplying (810) the preparation chamber (105) of the analysis unit (100) with a fluid to be prepared and/or with a reagent for performing a three-step first polymerase chain reaction in the preparation chamber (105) comprising the first subsidiary chamber (300), the second subsidiary chamber (305) and the third subsidiary chamber (310);
introducing (815) a product of the first polymerase chain reaction into the evaluation structure (110) of the analysis unit (100); and
evaluating (820) a two-step second polymerase chain reaction, performed in the segment chambers (115) and in the further segment chambers connected fluidically to these, using the product of the first polymerase chain reaction in the evaluation structure (110) and/or at least one product of the second polymerase chain reaction using the product of the first polymerase chain reaction.

10. Method (900) for producing an analysis unit (100) according to one of Claims 1 to 8, wherein the method (900) comprises the following steps:
providing (905) at least one lid element and a floor element, wherein the lid element has at least two lid recesses and the floor element has at least two floor recesses; and
formation (910) of a layered composite with at least one preparation chamber (105) for performing a three-step first polymerase chain reaction in order to obtain a reaction product, wherein the preparation chamber (105) is designed with a first subsidiary chamber (300) in a first temperature zone (345), a second subsidiary chamber (305) in a second temperature zone (350), and a third subsidiary chamber (310) in a third temperature zone (355), and at least one evaluation structure (110) for performing a two-step second polymerase chain reaction using the reaction product, wherein the formation (910) is effected by arranging the lid recesses opposite the floor recesses, wherein the evaluation structure (110) is or can be fluidically coupled to the preparation chamber (105), and wherein the evaluation structure (110) comprises at least two segment chambers (115), which are fluidically connected in parallel and are each fluidically connected respectively to a further segment chamber (400), and wherein the segment chambers (115) are arranged in the second temperature zone (350) and the further segment chambers (400) are arranged in the third temperature zone (355).

11. Control unit designed to perform, implement and/or drive all the steps of a method according to Claim 9.

12. Computer program designed to perform, implement and/or drive all the steps of a method according to Claim 9.

13. Machine-readable storage medium, with a computer program according to Claim 12 stored thereon.

## Revendications

1. Unité d'analyse (100) pour effectuer une réaction en chaîne de polymérase (200) imbriquée, l'unité d'analyse (100) présentant les caractéristiques suivantes :
au moins une chambre de préparation (105) pour effectuer une première réaction en chaîne de polymérase, afin d'obtenir un produit de réaction ; et
au moins une structure d'analyse (110) pour effectuer une deuxième réaction en chaîne de polymérase en utilisant le produit de réaction, la structure d'analyse (110) étant ou pouvant être accouplée fluidiquement à la chambre de préparation (105) et la structure d'analyse (110) comprenant au moins deux chambres segmentées (115) qui sont branchées en parallèle fluidiquement,
la chambre de préparation (105) étant réalisée avec une première chambre partielle (300), une deuxième chambre partielle (305) et une troisième chambre partielle (310), la première chambre partielle (300) étant disposée dans une première zone de température (345), la deuxième chambre partielle (305) étant disposée dans une deuxième zone de température (350) et la troisième chambre partielle (310) étant disposée dans une troisième zone de température (355) de l'unité d'analyse (100) pour effectuer une première réaction en chaîne de polymérase à trois étapes,
les au moins deux chambres segmentées (115) étant connectées fluidiquement chacune avec une chambre segmentée supplémentaire (400) pour une deuxième réaction en chaîne de polymérase à deux étapes, les chambres segmentées (115) étant connectées fluidiquement à la deuxième chambre partielle (305) et les chambres segmentées (115) étant disposées dans la deuxième zone de température (350) et les chambres segmentées supplémentaires (400) étant disposées dans la troisième zone de température (355).

2. Unité d'analyse (100) selon la revendication 1, **caractérisée en ce que** les chambres segmentées (115) présentent chacune au moins un réactif pour effectuer la deuxième réaction en chaîne de polymérase.

3. Unité d'analyse (100) selon la revendication 2, **caractérisée en ce que** les chambres segmentées (115) présentent chacune une autre quantité de réactif et/ou à chaque fois au moins un autre réactif.

4. Unité d'analyse (100) selon l'une quelconque des revendications précédentes, **caractérisée en ce qu'**une partie de la chambre de préparation (105) et une partie supplémentaire de la chambre de préparation (105) sont réalisées pour être amenées chacune à une autre température et/ou une partie de la structure d'analyse (110) et une partie supplémentaire de la structure d'analyse (110) sont réalisées pour être amenées chacune à une autre température et/ou une région de la chambre de préparation (105) et une région de la structure d'analyse (110) sont réalisées de manière à être amenées chacune à une autre température.

5. Unité d'analyse (100) selon l'une quelconque des revendications précédentes, **caractérisée en ce que** la structure d'analyse (110) comprend au moins deux chambres segmentées supplémentaires (400), à chaque fois l'une des chambres segmentées supplémentaires (400) étant branchée en série fluidiquement avec à chaque fois l'une des chambres segmentées (115).

6. Unité d'analyse (100) selon la revendication 5, **caractérisée en ce que** les chambres segmentées (115) et les chambres segmentées supplémentaires (400) sont réalisées pour être amenées chacune à une autre température.

7. Unité d'analyse (100) selon l'une quelconque des revendications précédentes, **caractérisée en ce que** la chambre de préparation (105) et la structure d'analyse (110) sont des composants d'une structure de canal pour commander un écoulement de fluide dans l'unité d'analyse (100), en particulier la structure de canal présentant au moins une soupape (342, 342), en particulier la soupape (340) étant réalisée pour désaccoupler fluidiquement la chambre de préparation (105) et la structure d'analyse (110).

8. Unité d'analyse (100) selon l'une quelconque des revendications précédentes, **caractérisée par** au moins une chambre de pompe (605) pour pomper un fluide à préparer dans la chambre de préparation (105) et/ou le produit de réaction hors de la chambre de préparation (105).

9. Procédé (800) pour faire fonctionner une unité d'analyse (100) selon l'une quelconque des revendications 1 à 8, le procédé (800) comprenant les étapes suivantes :
fourniture (805) de l'unité d'analyse (100) ;
sollicitation (810) de la chambre de préparation (105) de l'unité d'analyse (100) avec un fluide à préparer et/ou avec un réactif pour effectuer une première réaction en chaîne de polymérase à trois étapes dans la chambre de préparation (105) comprenant la première chambre partielle (300), la deuxième chambre partielle (305) et la troisième chambre partielle (310) ;
introduction (815) d'un produit de la première réaction en chaîne de polymérase dans la structure d'analyse (110) de l'unité d'analyse (100) ; et
analyse (820) d'une deuxième réaction en chaîne de polymérase à deux étapes effectuée dans les chambres segmentées (115) et les chambres segmentées supplémentaires connectées fluidiquement à celles-ci en utilisant le produit de la première réaction en chaîne de polymérase dans la structure d'analyse (110) et/ou d'au moins un produit de la deuxième réaction en chaîne de polymérase en utilisant le produit de la première réaction en chaîne de polymérase.

10. Procédé (900) de fabrication d'une unité d'analyse (100) selon l'une quelconque des revendications 1 à 8, le procédé (900) comprenant les étapes suivantes :
fourniture (905) d'au moins un élément de couvercle et d'un élément de fond, l'élément de couvercle présentant au moins deux évidements de couvercle et l'élément de fond présentant au moins deux évidements de fond ; et
formation (910) d'un composite stratifié comprenant au moins
une chambre de préparation (105) pour effectuer une première réaction en chaîne de polymérase à trois étapes afin obtenir un produit de réaction,
la chambre de préparation (105) étant réalisée avec une première chambre partielle (300) dans une première zone de température (345), une deuxième chambre partielle (305) dans une deuxième zone de température (350) et une troisième chambre partielle (310) dans une troisième zone de température (355),
et au moins une structure d'analyse (110) pour effectuer une deuxième réaction en chaîne de polymérase à deux étapes en utilisant le produit de réaction, la formation (910) s'effectuant en disposant les évidements de couvercle en regard des évidements de fond, la structure d'analyse (110) étant ou pouvant être accouplée fluidiquement à la chambre de préparation (105) et la structure d'analyse (110) comprenant au moins deux chambres segmentées (115) qui sont branchées en parallèle fluidiquement et qui sont connectées à chaque fois à une chambre segmentée supplémentaire (400), et les chambres segmentées (115) étant disposées dans la deuxième zone de température (350) et les chambres segmentées supplémentaires (400) étant disposées dans la troisième zone de température (355).

11. Unité de commande, réalisée pour effectuer, mettre en oeuvre et/ou commander toutes les étapes d'un procédé selon la revendication 9.

12. Programme informatique prévu pour effectuer, mettre en oeuvre et/ou commander toutes les étapes d'un procédé selon la revendication 9.

13. Support de mémoire lisible par machine comprenant un programme informatique selon la revendication 12 mémorisé sur celui-ci.
